# EUROPEAN PATENT APPLICATION

(11) **EP 4 807 688 A1**
(43) Date of publication of application: **16.09.2026**
(21) Application number: 25305323.5
(22) Date of filing: 12.03.2025
(51) Int. Cl.: G06V 10/25, G06V 10/26, G06V 10/44

(54) **PROCESSING A MEDICAL IMAGE OF A BODY OF A PATIENT ON A HORIZONTAL SUPPORT**

(71) Applicant: Dassault Systèmes, 78140 Vélizy-Villacoublay (FR)
(72) Inventor: SIMON, Aloïs, 78140 Vélizy-Villacoublay (FR)
(74) Representative: Bandpay & Greuter

(57) **Abstract**

The disclosure notably relates to a computer-implemented method for processing a medical image of a body of a patient on a horizontal support. The method comprises obtaining the medical image. The medical image comprises voxels. Each voxel is associated with a value. The value represents tissue density at the position represented by the voxel. The method further comprises discarding each voxel of the medical image associated with a value smaller than a predefined threshold. The discarding splits the image into connected components separated by discarded voxels. The method further comprises identifying, among the connected components, a connected component having a largest number of voxels. The method further comprises computing a curve separating the identified connected component from the remaining part of the medical image. The voxels bounded by the curve correspond to the body of the patient.

## Description

### TECHNICAL FIELD

The disclosure relates to the field of computer programs and systems, and more specifically to a method, system and program for processing a medical image of a body of a patient on a horizontal support.

### BACKGROUND

In the field of medical imaging, the presence of artifacts, such as the table on which the patient lies, patient supports or cushions, can significantly impede the analysis and interpretation of scans. The process of CT-scan acquisition can for example introduce significant artifacts into the imaging data, obscuring critical anatomical structures and leading to misinterpretations by radiologists or other practicians. While various algorithms and techniques have been developed to address these challenges, several existing solutions exhibit notable limitations. Such solutions include the following:
- **Traditional Image Segmentation Techniques:**
   ∘ **Thresholding and Region Growing:** These techniques rely on intensity values and local connectivity to distinguish between the patient and the table. *Region growing* begins by selecting one or more seed points in the image, typically chosen based on known intensity characteristics of the target region (e.g., the table). Starting from these seeds, the algorithm iteratively adds neighboring voxels to the region if they satisfy predefined criteria, such as intensity similarity and spatial connectivity. However, they are highly sensitive to noise and variations in imaging conditions, often resulting in incomplete removal of the table or inclusion of patient data.
   ∘ **Drawback:** These methods may not perform well in cases where the table has similar intensity values to the anatomical structures, leading to misclassification. These operations can introduce artifacts or lead to the loss of small anatomical details, compromising diagnostic quality.
- **Deep Learning Approaches:**
   ∘ Recent advancements have employed convolutional neural networks (CNNs) for image segmentation, including the removal of unwanted structures like tables. While these methods show promise, they often require large annotated datasets and significant computational resources for training.
   ∘ **Drawback:** The dependency on large datasets can be a barrier, especially in scenarios where annotated data is scarce (which is typically the case in the medical context, due to confidentiality constraints). Additionally, generalization across different imaging modalities and protocols remains a challenge.

Notably, existing segmentation techniques often struggle to accurately differentiate between the non-human body artifacts and the patient's anatomy due to similar intensity values in the images. This can result in incomplete removal of the artifact and the potential inclusion of patient data. Besides, many current methods require manual intervention or adjustment by radiologists or technicians to achieve satisfactory results, making the process time-consuming and possibly subjective.

There is thus a need for improved solutions for processing a medical image of a body of a patient on a horizontal support.

### SUMMARY

It is therefore provided a computer-implemented method for processing a medical image of a body of a patient on a horizontal support. The method comprises obtaining the medical image. The medical image comprises voxels. Each voxel is associated with a value. The value represents tissue density at the position represented by the voxel. The method further comprises discarding each voxel of the medical image associated with a value smaller than a predefined threshold. The discarding splits the image into connected components separated by discarded voxels. The method further comprises identifying, among the connected components, a connected component having a largest number of voxels. The method further comprises computing a curve separating the identified connected component from the remaining part of the medical image. The voxels bounded by the curve correspond to the body of the patient.

The method may comprise one or more of the following:
- computing the curve comprises:
   ∘ projecting the medical image along an axial plane defined by a Left-Posterior-Superior (LPS) or Right-Anterior-Superior (RAS) coordinate system of the medical image; and
   ∘ computing a 2D curve separating the projection of the identified connected component from the remaining part of the projected medical image;
- computing the 2D curve comprises performing an interpolation based on extreme points of the projection of the connected components;
- the image is a CT-scan and the value representing tissue density is a Hounsfield Unit (HU) value,
- the threshold is comprised between -90HU and -110HU, for example equal to -100HU; and/or
- the obtained medical image comprises at least one artefact.

It is further provided a computer program comprising instructions for performing the method.

It is further provided a computer readable storage medium having recorded thereon the computer program.

It is further provided a system comprising a processor coupled to a memory (and optionally to a graphical user interface), the memory having recorded thereon the computer program.

### BRIEF DESCRIPTION OF THE DRAWINGS

Non-limiting examples will now be described in reference to the accompanying drawings, where:
- FIG.s 1 to 8 illustrate the method; and

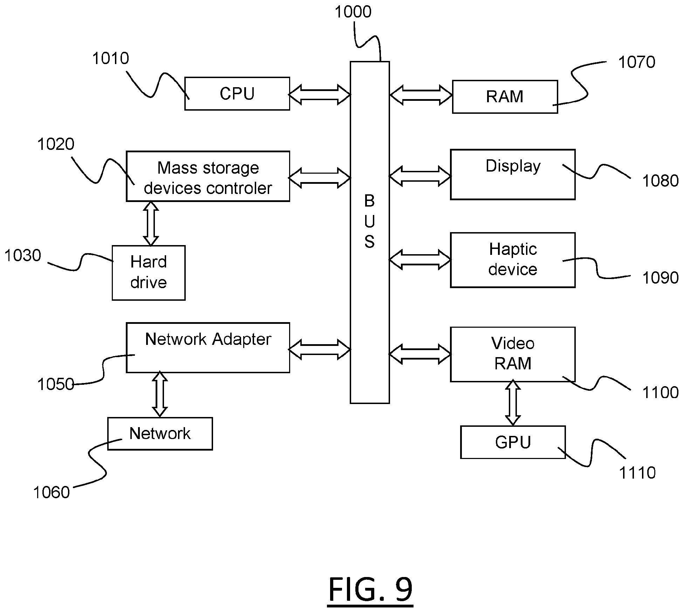

- FIG. 9 shows an example of the system.

### DETAILED DESCRIPTION

It is proposed a computer-implemented method for processing a medical image of a body of a patient on a horizontal support. The method comprises obtaining the medical image. The medical image comprises voxels. Each voxel is associated with a value. The value represents tissue density at the position represented by the voxel. The method further comprises discarding each voxel of the medical image associated with a value smaller than a predefined threshold. The discarding splits the image into connected components separated by discarded voxels. The method further comprises identifying, among the connected components, a connected component having a largest number of voxels. The method further comprises computing a curve separating the identified connected component from the remaining part of the medical image. The voxels bounded by the curve correspond to the body of the patient.

The method constitutes an improved solution for processing a medical image of a body of a patient on a horizontal support.

First, the method removes artefacts on the medical image of the body of the patient on the horizontal support (a table, e.g. for a CT-scan, or the like). Indeed, the method determines the voxels of the image that correspond to the body of the patient, i.e. only or substantially only the voxels representing the body, without the voxels that represent the support (e.g. a table, or the like) or other artefacts (cushions, clothes of the patient, or the like). For that, the method discards the voxels having a value (representing tissue density) under a threshold, because these voxels normally do not correspond to the body of the patient. To be more specific, it may happen that some voxels which are part of the patient body position are discarded, but these are isolated voxels: the voxels corresponding to the body are, in a large part (*i*.*e*. aside for few exceptions), non-discarded, whereas the voxels corresponding to the remaining parts of the scene (*e.g.* the support) are, in a large part (*i.e.* aside from possible few exceptions), discarded. This discarding splits the image into connected components formed by groups of neighboring voxels separated by discarded ones, and the method identifies the biggest of these components as the patient body (the patient body on the table or similar horizontal support necessarily corresponds to the biggest component in the scene captured by the image, simply because the body is with certainty the largest object that can be seen in the image). The method then computes a curve that bounds this component and the voxels within this curve (including inside voxels that could have been discarded, due for example to artefacts) form the whole set of voxels that represent the body. The method thus processes the medical image and finds/outputs its voxels that correspond to the patient body, thereby having eliminated the support and any artefact (cushion, clothes, or the like).

Furthermore, not only does the method achieve this result, but it does so in an improved manner. The method streamlines the artifact removal process, significantly reducing the time required for image processing. Unlike region growing in 3D Slicer (as described previously), which may require extensive manual adjustments, the method offers an automated approach (all the method steps may be carried out automatically by the computer system), which allows for quicker workflow integration. The method may in particular be implemented in web-based applications, where it is particularly efficient for web-based application and may be used in local on the client computer of the end user computer, which is not the case in many application (deep learning approach often need a remote to process the image) and algorithm like region-growing in 3D can be slow on the user computer if it is implemented in a web application.

The method is for processing a medical image of a body of a patient on a horizontal support. In other words, the method takes as input the medical image and processes it. Specifically, the processing consists in the method steps, which result in the curve determined by the method and the voxels enclosed within this curve. The method may output these voxels.

The medical image is an image of the body of the patient (*i*.*e*. human) on a horizontal support. "Image of the body" means that the image captures either the whole body of the patient, or only a part thereof. The image is an image capturing the body or at least a part thereof on a horizontal support. The horizontal support is any horizontal support on which a medical imaging process yielding the image can be done. The horizontal support is thus typically a table on which the patient lies during the imaging process (*e*.*g*. CT-scan or MRI). The horizontal support may nevertheless be any other possible horizontal support on which medical imagery can be done, such as, for example, a support on which a patient's arm or leg rests for a partial imagery of only that arm or leg.

The method comprises obtaining the medical image. Obtaining the medical image may comprise performing a medical imagery process (for example a CT-scan if the image is a CT scan image) to obtain/acquire the image of the patient's body resting on the support. Alternatively, obtaining the medical image may comprise retrieving (*e*.*g*. downloading) the image from a (*e*.*g*. remote) memory or database or server where the image has been stored further to its acquisition by the medical imagery process. The obtained medical image may comprise at least one artefact (*i*.*e*. besides the support), *e*.*g*. a cushion or a piece of clothing on the support.

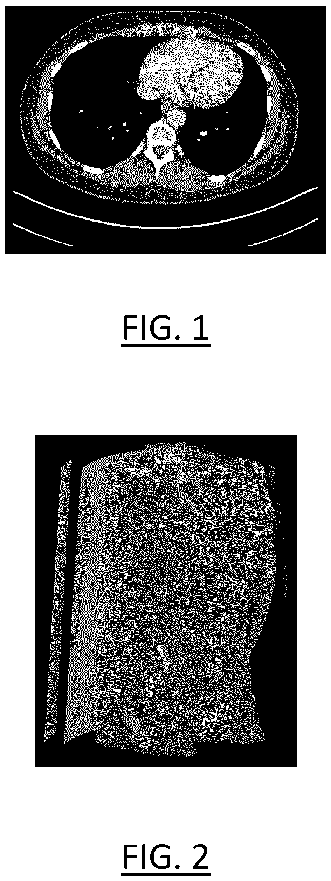

The image comprises voxels, *i*.*e*. the image is formed by a collection of voxels each representing a respective position in the 3D scene (of the body resting on the support) captured by the image. Each voxel is associated with a value, which represents tissue density at the position represented by the voxel. In other words, the value associated with the voxel represents the density of tissue at the position which the voxel represents. The image may for example be a CT-scan, in which case the value representing tissue density is a Hounsfield Unit (HU value), *i*.*e*. each voxel is associated with a HU value capturing the tissue density at the position represented by the voxel. HU is a quantitative scale for describing radiodensity in CT imaging, where each voxel is assigned a value representing tissue density relative to water (*i*.*e*. the HU value represents tissue density relative to water). FIG. 1 shows a 2D view of an example of a CT scan image, and FIG. 2 shows an example of a complete CT-scan image (thus in 3D). The method may comprise computing the value of tissue density for each voxel in the image. Alternatively, these values may already be computed during acquisition of the image by the medical imaging process, as part of the imaging process (for example HU value may be automatically computed during a CT-scan, or as an automated post-processing step following the image acquisition of the CT-scan).

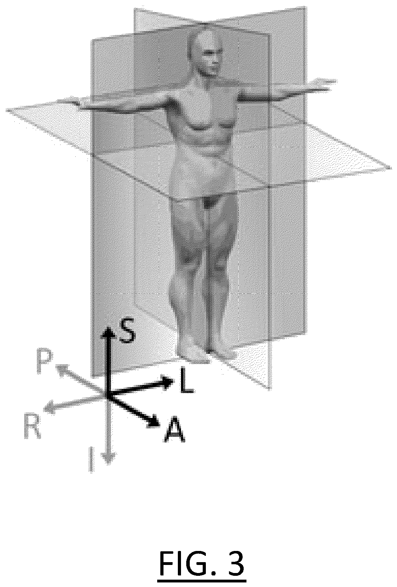

The medical image is a 3D image and may be associated with a coordinate system. The coordinate system may be the Left-Posterior-Superior (LPS) coordinate system or Right-Anterior-Superior (RAS) coordinate system. LPS (Left-Posterior-Superior) coordinate system is a standard 3D coordinate system in medical imaging where the origin is oriented along the left, posterior, and superior directions of the patient. RAS (Right-Anterior-Superior) coordinate system is an alternative 3D coordinate system in medical imaging where the origin is oriented along the right, anterior, and superior directions of the patient. FIG. 3 illustrate these two different coordinate systems.

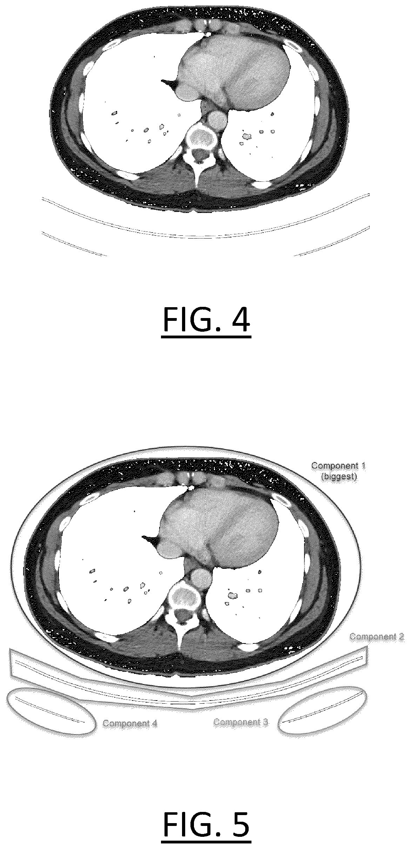

The method further comprises discarding each voxel of the medical image associated with a value smaller than a predefined threshold. The predefined threshold may be set by a user at an initial stage of the method or may be fixed and non-editable (and for example dependent on the type of image). For example, if the image is a CT-scan (or CT-scan image), the threshold may be comprised between - 90HU and -110HU, for example comprised between -95HU and -105HU, for example equal to -100HU (which corresponds to fat tissue). If the image is a PET ("Positron Emission Tomography") image, the value may be a SUV value ("Standardized Uptake Value"), and the threshold may be comprised between 1 and 30 SUV. If the image is an MRI image, the threshold may be dependent on the MRI type. Discarding may for example comprise browsing the voxels (*i*.*e*. going through a list of all the voxels) and discarding each voxel having value smaller than the threshold. For each discarded voxel, discarding the voxel may consist in setting its values to a predefined neutral value. FIG. 4 illustrates the result of the discarding applied to the example of FIG. 1: all the discarded voxels are shown in white. As can be seen on FIG. 4, the discarded voxels comprise the lungs, the inner part of the table and some voxels of the fatty tissues, which are all set to the neutral value.

The discarding splits the image into connected components separated by discarded voxels. Indeed, the thresholded image (*i*.*e*. the image after the discarding step) is a set of groups of connected voxels. Two voxels are connected if they are next to each other and if both values are not equal to the neutral value. Each such group is one said connected component.

The method further comprises identifying, among the connected components, a connected component having a largest number of voxels, *i.e.* the connected component, or group of connected voxels, having the largest number of voxels. This group necessarily corresponds to the human body, as the human body is necessarily the biggest component of the image, as previously explained. This may be done in any suitable manner. For example, the identification of the largest component may comprise forming a graph, where each voxel with a value different form the neutral value is a node and two nodes are connected if two corresponding voxels are connected. Then, the identification of the biggest component may comprise finding the largest connected component of that graph by using a suitable algorithm. Different algorithms exist to find the biggest component of a graph, such as the "depth-first search" algorithm to explore the graph and find the components. The method may use any of these known algorithms, which algorithm is chosen being a matter of implementation. FIG. 5 illustrates the various components of the example shown in FIG. 4.

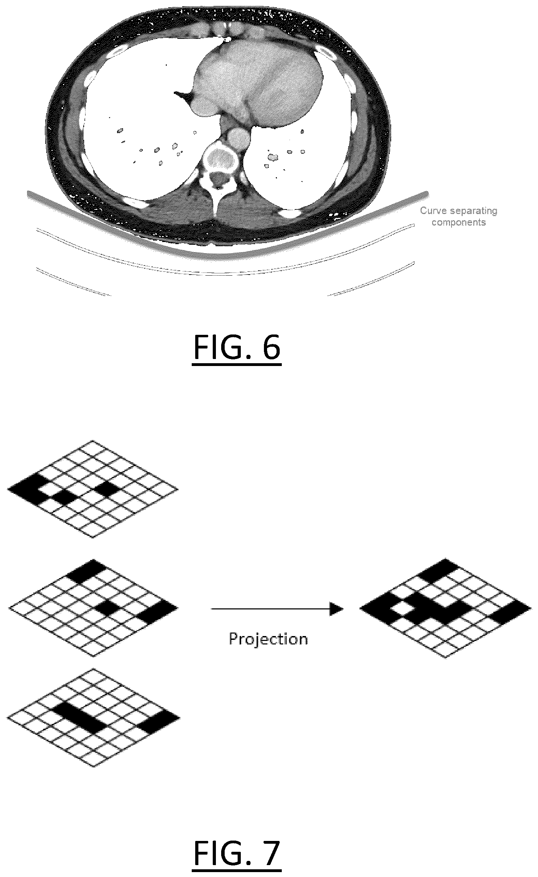

The method further comprises computing a curve separating the identified connected component from the remaining part of the medical image (i.e. from the other voxels of the medical image). The voxels bounded by the curve (i.e. on the same side of the curve as the side on which the voxels of the largest connected component are) correspond to the body of the patient, *i.e.* these voxels form the part of the image that captures the body of the patient (and not the support nor any artefact). These voxels of course include the voxels of the largest connected components but may also include voxels that were discarded at the discarding step even though they represent body portions. FIG. 6 illustrates the curve computed for the example of FIG.s 4-5. It is to be understood that FIG.s 1 and 4 to 6 show 2D views of the CT-scan image for the sake of illustration of the method steps: the method is however performed for the complete 3D CT-scan image (like the one shown in FIG. 2).

Computing the curve may comprise projecting the medical image (*i.e.* all the voxels of the image) along an axial plane defined by a Left-Posterior-Superior (LPS) or Right-Anterior-Superior (RAS) coordinate system of the medical image. The axial plane may be the plane defined by the left-right axis and the anterior-posterior axis in the coordinate system (LPS or RAS), i.e. the plane generated by the span of the unit/basis vectors of these two axes. This allows to separate the identified biggest connected component from the other components. The projection results in a single 2D image where the non-neutral values (the values of the projected non-discarded voxels) represent the components. FIG. 7 illustrates the projection.

Computing the curve may then comprise computing a 2D curve separating the projection of the identified (largest) connected component from the remaining part of the projected medical image, *i.e.* computing the equation of a 2D curve to separate the projected components. This may comprise performing an interpolation based on extreme points of the projection of the connected components. This may be done using known methods for that purpose, such as the quadratic interpolation (for a parabola) or the three-point circle equation (for a circular arc). The method may for example take the voxel with the highest coordinate on the Left-Right axis, the one with the lowest coordinate on the Left-Right axis and a third with the lowest coordinate on the Posterior-Anterior axis and compute the equation of a curve passing by those three points. This curve will separate the components along the Left-Right axis. Alternative interpolations may be used, this is a matter of implementation.

The method may then comprise determining the plane or 2D surface of which projection on the axial plane yields the curve (for example by applying the inverse of the projection to the curve). This plane or surface may be referred to as "separating plane" or "separating surface. An alternative and simpler manner of finding the separating plane or surface, and which the method may implement, may be to extrude the curve along the plane defined by the axes right-left and superior-inferior. The method may then keep all the voxels which have a coordinate on the anterior-posterior axis below the separating plane or separating surface, which are the said voxels bounded by the curve (*i.e.* the voxels of which projection is bounded by the curve and which are on the same side of the curve as the projection of the voxels of the biggest component). These voxels form the complete set of voxels that represents the body on the image, without artefacts, and without the support.

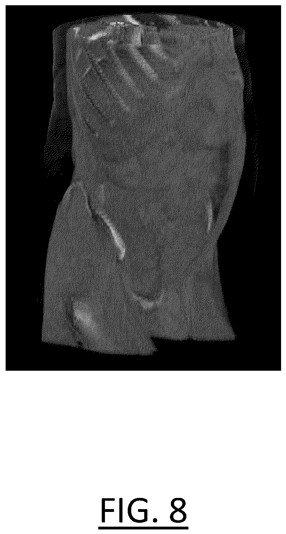

FIG. 8 shows the voxels of the body, without the table or other artefacts, outputted by the method for the example of CT-scan image of FIG. 1.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

FIG. 9 shows an example of the system, wherein the system is a client computer system, e.g. a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

## Claims

1. A computer-implemented method for processing a medical image of a body of a patient on a horizontal support, the method comprising:
- obtaining the medical image, the medical image comprising voxels each being associated with a value representing tissue density at the position represented by the voxel;
- discarding each voxel of the medical image associated with a value smaller than a predefined threshold, the discarding splitting the image into connected components separated by discarded voxels;
- identifying, among the connected components, a connected component having a largest number of voxels; and
- computing a curve separating the identified connected component from the remaining part of the medical image, the voxels bounded by the curve corresponding to the body of the patient.

2. The method of claim 1, wherein computing the curve comprises:
- projecting the medical image along an axial plane defined by a Left-Posterior-Superior (LPS) or Right-Anterior-Superior (RAS) coordinate system of the medical image; and
- computing a 2D curve separating the projection of the identified connected component from the remaining part of the projected medical image.

3. The method of claim 2, wherein computing the 2D curve comprises performing an interpolation based on extreme points of the projection of the connected components.

4. The method of any one of claims 1 to 3, wherein the image is a CT-scan and the value representing tissue density is a Hounsfield Unit (HU) value.

5. The method of claim 4, wherein the threshold is comprised between -90HU and - 110HU, for example equal to -100HU.

6. The method of any one of claims 1 to 5, wherein the obtained medical image comprises at least one artefact.

7. A computer program comprising instructions which, when executed by a computer system, cause the system to perform the method of any one of claims 1 to 6.

8. A computer-readable data storage medium having recorded thereon the computer program of claim 7.

9. A computer system comprising a processor coupled to a memory, the memory having recorded thereon the computer program of claim 7.
